# EUROPEAN PATENT APPLICATION

(11) **EP 2 368 591 A1**
(43) Date of publication of application: **28.09.2011**
(21) Application number: 10157208.9
(22) Date of filing: 22.03.2010
(51) Int. Cl.: A61M 5/00

(54) **Container for needle assemblies for a drug delivery device**

(71) Applicant: Sanofi-Aventis Deutschland GmbH, 65929 Frankfurt am Main (DE)
(72) Inventor: Hamm, German F., 65926 Frankfurt am Main (DE)

(57) **Abstract**

The present invention relates to a container adapted to receive a number of disposable needle assemblies (16) for a drug delivery device. The container comprises:
- a body (12) of substantially cylindrical geometry adapted to receive at least the needle assemblies (16),
- at least one removable cap (14) releasably disposed at an open end section of the body (12),
- wherein the inner diameter of the body (12) matches with the outer diameter of the needle assembly (16).

## Description

The present invention relates to a container for needle assemblies like hypodermic injection needles to be used with a respective drug delivery device, such like a pen-type injector.

### Background and Prior Art

Drug delivery devices allowing for multiple dosing of a required dosage of a liquid medicinal product, such as liquid drugs, and further providing administration of the liquid to a patient, are as such well-known in the art. Generally, such devices have substantially the same purpose as that of an ordinary syringe.

Drug delivery devices of this kind have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose. In order to meet these requirements, the number of parts and steps required to assemble the device and an overall number of material types the device is made from have to be kept to a minimum.

Typically, the medicinal product to be administered is provided in a cartridge that has a moveable piston or bung mechanically interacting with a piston rod of a drive mechanism of the drug delivery device. By applying thrust to the piston in distal direction, a certain amount of the medicinal fluid is expelled from the cartridge. For injection a medicinal product, a disposable hypodermic needle is coupled with a product-containing cartridge in a fluid transferring way. Typically, the needle assembly can be mounted onto a distal end section of a cartridge holder of the drug delivery device. By way of mounting the needle assembly onto the cartridge holder, a proximal end section of the needle may penetrate a pierceable septum of the cartridge, thus establishing access to the medicinal product contained in the cartridge.

A drive mechanism operably engaged with a piston sealing the proximal end of the cartridge is adapted to exert pressure on the piston in order to translationally displace the piston in distal direction. This way, a pre-defined dose of the medicinal product can be expelled from the cartridge through the needle assembly and can be delivered into biological tissue.

After termination of the injection process, the needle is removed from the tissue. Subsequently, the needle assembly which is to be released and removed from the cartridge holder is discarded. For a subsequent dispensing procedure, a new needle assembly has to be used.

Even though such needle assemblies are separately packaged and sealed for hygienic reasons, they are commercially distributed in a loose form. For transportation of needles, patients make most commonly use of small bags, that are adapted to receive numerous of such needle assemblies in a loose and disordered way.

Depending on environmental conditions and depending on the treatment of such bags, a needle assembly may become subject to damage, for example when exposed to external forces or mechanical shock and vibration. It may occur, that needle assemblies clinch together and may therefore mutually destroy or damage a protective sheath or package.

### Objects of the Invention

It is therefore an object of the present invention, to provide an improved storage and transportation means for needle assemblies being adapted for drug delivery devices. In a further object, the needle assembly should be stored and transported in a space-saving way. It is a further object, to improve handling of a drug delivery device and it components.

### Summary of the Invention

The present invention provides a container adapted to receive a number of disposable needle assemblies for a drug delivery device. The container comprises a body that can have a preferably substantially cylindrical outer geometry which is adapted to receive the needle assemblies. The container or container unit further comprises at least one removable cap releasably disposed at an open end section of the body. By way of the removable cap, the container can be closed or sealed and the needle assemblies contained therein can be better protected against environmental impact.

Furthermore, the inner diameter of the housing, hence the substantially circular cross section of the housing matches with the outer diameter or with a respective cross section of the needle assembly.

Preferably, the needle assemblies are placeable or disposable in the housing only along a preferential direction which is governed by the overall geometry and dimensions of the housing of the container unit. Since the needle assembly typically has a cupped receptacle of substantially cylindrical shape and thus at least partially comprises a circular cross section, the radial dimensions of the cylindrical housing is chosen such, that a needle assembly can only be inserted into the housing along its longitudinal direction, wherein a needle portion of the needle assembly protruding from the needle assembly's receptacle is oriented substantially parallel to the longitudinal axis of the container and its housing.

In this way, the geometry and shape of the housing constrains the needle assemblies to be arranged in a preferred and pre-defined orientation.

Inner diameter of the housing may further be chosen in such a way, that the needle assemblies are at least frictionally engaged in the housing. This way, even externally applied mechanical vibrations or shocks would no longer lead to a mutual clinching of adjacently located needle assemblies. Hence, a danger of damage can be effectively reduced.

Preferably, the needle assemblies are arranged stack-like or in a nested way inside the housing. This way, a particular space-saving arrangement can be realized by the cylindrical housing.

In a further preferred aspect, both end sections of the container are closed by removable caps. Hence, the tubular shaped body can be opened at both of its end sections. Insertion or removal of needle assemblies stacked in the body can be facilitated. For instance, by removing both caps from the body, a needle assembly can be removed from the body at its distal end by inserting some kind of pushing means into the other, proximal end section of the tubular body. In this way, even needle assemblies being frictionally engaged with the inner wall of the body can be easily removed from the container by way of being pushed there through.

In a further preferred embodiment, the outer diameter of the container is substantially equal to the outer diameter of a drug delivery device of pen-injector type. Preferably, the outer diameter of the body almost exactly matches with the diameter of the housing of a particular drug delivery device which is to be coupled with the particular type of needle assembly stacked in the container.

Preferably and according to another beneficial aspect of the invention, also the axial extension of the container is within the range of an axial extension of said drug delivery device. By adapting the outer dimension of the container and its body to the outer dimensions of a corresponding drug delivery device, the container may substitute one of a plurality of drug delivery devices to be arranged in a carry case or travel kit.

This way, a fastening means originally intended for a drug delivery device can be used for the container instead. Consequently, if one of a plurality of fastening or fixing slots of a carrying or storage unit for drug delivery devices is not used for a drug delivery device, the container can be placed therein instead.

According to a further preferred embodiment, the body of the container is at least partially transparent. Therefore, at least a portion of the body is at least partially or fully transparent and/or the entire body is made of an at least partially transparent material. Preferably, the body comprises a plastic material, for instance a polymeric and/or thermoplastic material. Moreover, the body and/or its end caps are manufactured by way of a one- or multi-component injection molding process.

By providing an at least partially transparent body, the end-user may easily visually inspect the number of needle assemblies stored in the container.

In another preferred embodiment, the container is equipped with a pre-defined number of needle assemblies. Additionally, the needle assemblies may be stacked in the container by the supplier, thereby providing a high package density leading to a more effective use of storage space.

In another aspect, the invention also provides a carry case or carry bag for a drug delivery device comprising at least a first and a second slot, wherein each of said slots is adapted to releasably receive and to fix a drug delivery device like a pen-type injector. Moreover, the carry case is further equipped with an above-described container, which is releasably disposed in the first or second slot.

Even though a carry case may be intentionally designed to house two or even more injection pens, in circumstances, where only one or a reduced number of injection pens is actually in use, a free fixing slot can be universally used to hold a container equipped and filled with a stack of needle assemblies.

Preferably, the at least first and/or second slots comprise at least one fixing clip adapted to positively engage with the container and/or with a respective drug delivery device.

It will be apparent to those skilled in the pertinent art, that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following, preferred embodiments of the invention will be described in greater detail by making reference to the drawings in which:
- Figure 1: illustrates a carry case for receiving at least two drug delivery devices and
- Figure 2: schematically illustrates the container to be stacked with needle assemblies in an exploded perspective illustration.

### Detailed Description

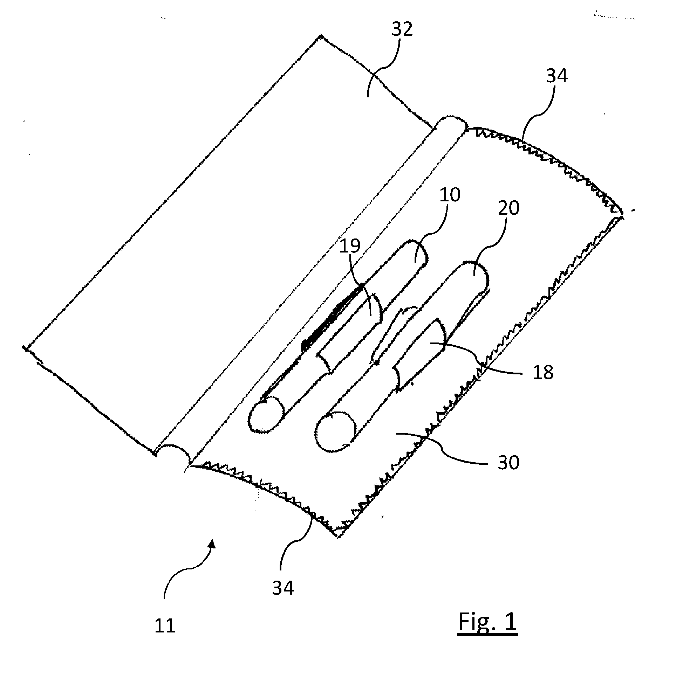

Figure 1 schematically illustrates a carry case 11 adapted to receive at least two drug delivery devices 20, e.g. a pen-type injector. In order to securely deposit the drug delivery devices 20 inside the carry case 11, the carry case 11 comprises two fixing slots 18, 19 in form of fixing clips, which are adapted to positively engage with the outer contour of the drug delivery device 20. The carry case 11 has a base portion 30 and a lid 32.

The lid 32 can be folded with respect to the base 30 in order to open or to close the carry case 11. For closing the carry case 11, in the illustrated embodiment, body 30 and lid 32 comprise mutually corresponding zipper means 34 at their outer circumference. Alternative to a zipper means 34, other commonly used closing mechanisms, like clips or snap fasteners can be universally applied here.

As illustrated in Figure 1, one of the fixing slots 18 is equipped with a drug delivery device 20 whereas the other fixing slot 19 is occupied with a container 10 according to the present invention.

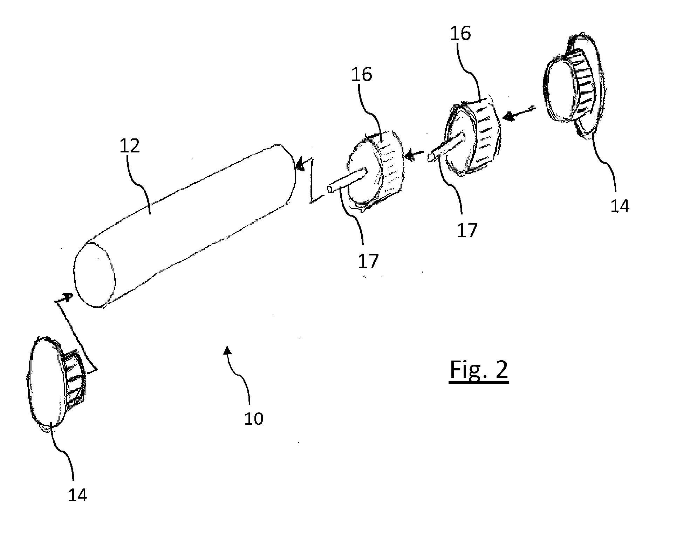

The container 10 is illustrated separately in Figure 2. The container 10 comprises a tubular body 12 adapted to receive a stack of needle assemblies 16. Here, a needle assembly comprises a cupped receptacle adapted to be engaged with a distal end section of a cartridge holder component of the injection device 20. In the centre of its receptacle portion, the needle assembly 16 comprises a protruding needle portion 17. In the illustration according to Figure 2, the various needle assemblies 16 are individually covered with a seal or a protective sheath.

The geometry of the tubular body 12 of the container and the individual needle assemblies 16 is preferably chosen such, that the needle assemblies 16 can only be inserted into the body 12 along a predefined preferential direction. Preferably, the needle assembly's circumferential sidewall section matches and/or abuts with the inner sidewall of the body 12. In this way even a frictional engagement of body 12 and needle assemblies 16 can be attained.

The body 12 can further be closed by means of two end caps 14 that can be releasably plugged or mounted to the open end of the tubular body 12. By removing both end caps 14, removal of a needle assembly 16 can be facilitated. For instance, by way of some pushing means, all needle assemblies 16 stacked inside the body 12 can be pushed in axial or longitudinal direction of the body 12 until an uppermost needle assembly 16 drops out of the body 12.

By making use of an at least partially transparent material for the body, the end user may easily check the number of needle assemblies 16 disposed inside the container 10. Moreover, the container 10 also provides general protection against environmental influences, like dust, dirt or moisture.

### List of Reference Numerals

- 10: container
- 11: carry case
- 12: body
- 14: end cap
- 16: needle assembly
- 17: needle section
- 18: fastening clip
- 19: fastening clip
- 20: drug delivery device
- 30: bottom section
- 32: flap
- 34: zipper

## Claims

1. A container adapted to receive a number of disposable needle assemblies (16) for a drug delivery device comprising:
- a body (12) of substantially cylindrical or other geometry adapted to receive at least the needle assemblies (16),
- at least one removable cap (14) releasably disposed at an open end section of the body (12),
- wherein the inner diameter of the body (12) matches with the outer diameter of the needle assembly (16).

2. The container according to claim 1, wherein the needle assemblies (12) are placeable in the body (10) only along a preferential direction governed by the geometry of the body (10).

3. The container according to any one of the preceding claims, wherein the long axis of the cylindrical container is substantially parallel to the direction of elongation of a needle section (17) of the needle assembly (16).

4. The container according to any one of the preceding claims, wherein both of the body's (12) end sections are closed by a removable cap (14).

5. The container according to any one of the preceding claims, wherein its outer diameter of the body (12) is substantially equal to the outer diameter of a drug delivery device (20), such like a pen-type injector.

6. The container according to any one of the preceding claims, wherein the axial extension of the body (12) is within the range of an axial extension of the drug delivery device.

7. The container according to any one of the preceding claims, wherein the body (12) is at least partially transparent.

8. The container according to any one of the preceding claims, wherein the body (12) is stacked with a pre-defined amount of needle assemblies (16).

9. A carry case for a drug delivery device comprising at least a first and a second slot (18, 19) each of which being adapted to releasably receive a drug delivery device (20) of pen-injector type, wherein a container (10) according to any one of the preceding claims is releasably disposed in the first or second slot (18, 19).

10. The carry case according to claim 9, further comprising a drug delivery device (30) of pen-injector type releasably disposed at the second or first slot (19, 18).

11. The carry case according to any one of the preceding claims 9 or 10, wherein first and /or second slots comprise at least one fixing clip (18, 19) adapted to positively engage with the container (10) and/or with the drug delivery device (20).
